# EUROPEAN PATENT APPLICATION

(11) **EP 1 880 762 A2**
(43) Date of publication of application: **23.01.2008**
(21) Application number: 06755323.0
(22) Date of filing: 03.05.2006
(51) Int. Cl.: B01J 31/02, A61K 9/50, A61K 9/51, A23P 1/04, B05B 7/06

(54) **METHOD OF PREPARING MICRO- AND NANOMETRIC PARTICLES WITH LABILE PRODUCTS**

(30) Priority: 04.05.2005 ES 200501107
(71) Applicant: Universidad de Sevilla, E-41012 Sevilla (ES)
(72) Inventor: GANAN CALVO, Alfonso Miguel, Camino De Los Descubrimientos s/n, 41092 Sevilla (ES); CHAVEZ DE DIEGO, Sebastian, Avda. Reina Mercedes s/n, 41012 Sevilla (ES); CEBOLLA RAMIREZ, Angel, Avda. Reina Mercedes s/n, 41012 Sevilla (ES); FLORES MOSQUERA, María, Camino De Los Descubrimientos s/n, 41092 Sevilla (ES); DE CASTRO HERNANDEZ, Elena, Camino De Los Descubrimientos s/n, 41092 Sevilla (ES)
(74) Representative: Elzaburu Marquez, Alberto
(86) International application number: PCT/ES2006/000212
(87) International publication number: WO 2006/117422

(57) **Abstract**

The hereby-present invention refers to the generation of polymeric particles in the micro and nanometric range with a controlled and reproducible method. Said particles have spherical shape and a very narrow and homogeneous distribution. Particularly, the present invention describes the use and application of a gentle method for the production of particles and its implementation to the encapsulation of fragile compounds of biological interest, including from peptides and proteins to cells and microorganisms.

## Description

### STATE OF THE ART

Currently, the use of microparticles has spread widely in fields such as pharmacy, biomedicine, cosmetic industry, food industry, agriculture, veterinary science, textile industry, chemistry, etc. Among others, the most interesting application is the possibility to use microparticles as a method to stabilize and protect products from the environment and/or as procedure to optimize the distribution of an encapsulated compound in its way to the application/interaction point keeping its activity.

The use of microparticles acquires more importance when the compounds used, such as labile molecules, peptides and proteins, cells, microorganisms, etc., are unstable or require specific conditions to keep their viability during their complete procedure including formulation, production, distribution, storage and release process. Therefore, immobilizing labile compounds inside polymeric matrixes is a methodology with an increase used in medical research, pharmaceutical research, bioengineering research, food industry research, etc. The size of the particle is crucial in order to determine the possibility to be used in some of these fields, i.e. food industry, development of new drugs, cells encapsulation, etc.

The different methods for microencapsulation can be classified in three large groups: physicochemical, chemical and mechanical. The microencapsulation method is chosen depending on the coating material and the physicochemical properties of the active principle. Nevertheless, there isn't a fixed procedure for one material and one active principle. In several cases it is possible to choose among several possibilities (S. Gouin, "Microencapsulation: industrial appraisal of existing technologies", Trends Food. Sci Technol. 2004; V. R. Sinha, A. Trehan, "Biodegradable microspheres for protein delivery", J. Control. Rel. 2003, 90, 261-280). Generally, the most habitual procedures for encapsulation of fragile compounds are based on techniques including ionic emulsification-gelation of the encapsulating material, and/or atomization of the mixture by means of extrusion of the mixture through capillary. Nevertheless, these methods have problems not yet solved. Among them we should emphasize the low viability or final activity of the encapsulated compounds due to the aggressive techniques usually applied (Brynjelsen S. et al, US6835396 "Preparation of submicron sized nanoparticles via dispersion lyophilization"; Kyekyoon K. et al, US5344676, "Method and apparatus for producing nanodrops and nanoparticles and thin film deposits therefrom"); and the impossibility to produce monodipersed particles of small size (d ≤ 300 µm), since size is given by the diameter of the atomization capillary, usually big enough to prevent clogging (Pacifico C.J. et al, W00145835, "Sensitive substance encapsulation"; Pluess-Wenzinge R. et. al, WO9944735, "Method and device for capsuling microbial, plant and animal cells or biological and chemical substances").

Therefore, the possibility to have microparticle production methods combining both control of the particle size and non-aggressive treatment of molecules with a biological nature would mean a big advance in the development of microencapsulation of labile compounds.

### DESCRIPTION OF THE INVENTION

The hereby-presented invention refers to the application of a method for production of particles containing labile compounds by means of a Flow Focusing system, which combines the application of hydrodynamic forces and a specific geometry of the system to generate drops with the required properties and, solidification of the drops in order to obtain particles of nano and micrometric size, with spherical shape and a very narrow and reproducible size distribution.

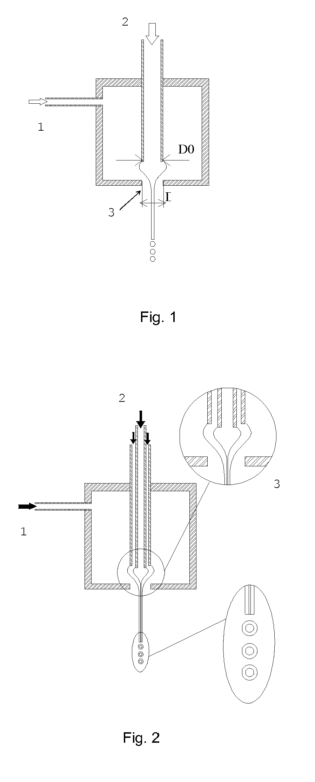

The system for production of particles referred to by this invention is constituted by a Flow Focusing device which geometry is shown in Fig. 1. This device consists of a chamber pressurized by means of the continuous supply of a fluid (1), which is expelled outside through the one single exit orifice in the chamber (D). Inside the chamber one or more fluids (2) are injected through a feeding point (D0) placed in front of the one single exit hole of the chamber. The fluid flow pressurizing the chamber surrounds the second fluid injected through D0 and expels it outside the chamber through the hole producing a thin microjet in a controlled way.

The microjet is inside a laminar flow, due to capillary instabilities the microjet breaks, giving rise to a drops distribution with a controlled size. Once the drops have been produced, they undergo a drying and/or solidification process, which gives rise to obtaining dry particles with spherical shape and a homogeneous size distribution equal to the size distribution of the drops produced initially. In this procedure, the described fluids are liquids or gases. When two or more liquids are used simultaneously, they must be different enough between themselves to allow the generation of a stable microjet. Generally, the inner fluid is a solution of one or more components, a liquidized solid, a suspension and/or an emulsion of compounds of diverse nature.

In the first procedure, one single fluid is injected through one single feeding tube inside the chamber pressurized by the focussing fluid, and is then expelled to the outside through a hole placed in the wall of the chamber in front of the end of the feeding tube.

In the second procedure included in this invention, the injected fluid consists of different fluids, which are introduced through concentric capillary tubes. Inside the chamber, these fluids get in touch creating a capillary microjet formed by several layers of concentric fluids. This capillary microjet is pressurized and expelled outside the camera by the liquid pressurizing the chamber through the hole placed in the wall in front of the end of the feeding tubes.

It is aim of this invention the use of devices containing multiple fluid feeding tubes inside the pressurized chamber, producing multiple microjets that exit to the outside through the holes made in front of each feeding tube, allowing obtain particles in big amounts.

As an option, it is included in this invention the possibility to apply periodical and controlled external instabilities (e.g. mechanical, acoustic, etc.) to one or more fluids, in order to enhance even more the production of particles with a homogeneous size distribution.

It is the aim of this invention to provide a new method for encapsulation of labile molecules, peptides and proteins, DNA, biologically active compounds, cells, microorganisms, etc., by means of a very gentle working conditions allowing both feasibility and final functionality of the encapsulated compound. As well, it is the aim of this invention the use of a procedure by means of which there is obtained dry particles with a predictable and controlled size with a low dispersion.

Also it is included in this invention a procedure for the production of particles that has molecules of biological interest expose to the environment.

Main advantageous characteristics that allow the use of this technology, which combines geometry and hydrodynamic effect, for encapsulation of fragile compounds are:
a) There is no contact between internal fluid and exit orifice, so that it is avoided the problems of the big shear stresses related with direct extrusion. Problems of clogging are avoided.
b) It is possible to produce particles smaller than any other dimension of the device (orifice, feeding tube, etc.), it is not possible with other technologies of extrusion, which imposes the size by means the injection orifice.
c) The particle size is controllable and reproducible.
d) The size distribution of the particles is very narrow, without need to resort to other more complex mechanism of microjet excitation that could affect the encapsulated compound, and without need to make a subsequent selection.
e) It is a totally reproducible process.
f) It is possible to use several liquid combinations with different physical-chemical properties (viscosity, chemical compound, etc.)
g) The final composition and the structure of the particles is controllable from the beginning of the procedure.
h) It is a continuous system for production of particles, which allows carrying out exhaustive quality controls in each step of the production process.

### DESCRIPTION OF THE FIGURES

Figure 1: general scheme with basic components of a device for the generation of particles by means of injection of one single fluid according to the hereby described procedure: (1) Focusing fluid, (2) focused fluids, (3) meniscus.
Figure 2: general scheme with basic components of a device for the generation of particles by means of the injection of two concentric fluids: (1) Focusing fluid, (2) focused fluids, (3) meniscus.
Figure 3a: Microscope photograph of particles of alginate with BSA-Flu.
Figure 3b: fluorescence microscope image of alginate particles with BSA without any dye.
Figure 3c: fluorescence microscope image of alginate particles with BSA with fluorescein.
Figure 4a: fluorescence microscope image of alginate particles.
Figure 4b: microscope photograph of alginate particles with GFP.
Figure 4c: fluorescence microscope image of alginate particles with GFP from Fig. 4b.
Figure 5a: fluorescence microscope image of pyrene particles.
Figure 5b: fluorescence microscope image of polystyrene particles with BSA with fluorescein.
Figure 6a-c: fluorescence microscope images of alginate microparticles with different concentrations of a modified GFP producer E. Coli strain : a) 2·10⁸ cfu/gr, b) 8·10⁸ cfu/gr, c) 1,3·10⁹ cfu/gr.

### DETAILED DESCRIPTION OF THE INVENTION

Prior to the detailed description of the invention, we must highlight that this invention is not restricted neither to the specific components nor to the concrete procedures hereby described, which may obviously vary. In general, it is to be understood that all terminology used here, is used to describe the different aspects of the invention and does not intend to be in any case limiting.

Considering the characteristics of Flow Focussing technology, new procedures have been developed using devices described in the following documents, among which are included: W09743048 ("Liquid atomization process"), W09930833 ("Device and method for creating dry particles"), W09930833 ("Device and method for creating aerosols for drug delivery"), WO03066231 ("Device for the production of capillary jets and micro- and nanometric particles") US6234402 ("Stabilized capillary microjet and devices and methods for producing the same"), P200500205 ("Procedure and device for the production of particles of micro and nanometric size").

In general and except where stated, all scientific and technical terms used herein this document have the same meaning that is usually understood when applied in related environments.

### Definitions

It must be noted that as used herein and in the appended claims the singular forms "a", "one" and "the", can also indicate plurals unless the context indicates otherwise. For example, when it is written "a particle" it includes a group of particles and when it is referred to "a compound" it includes a combination of compounds, etc.

In this invention the terms *sphere, particles and capsules* are interchangeable for the description of the micro and nanoparticles described in the patent, regardless of whether they are solid, hollow, porous, with different layers o multi-layer, etc. These particles can be made of any material depending on the final application.

With the term *reactive surface* we refer to the surface of the particles that may get in touch with the environment, including the external surface of the sphere, the pores and the channels created, and that, having any composition, have a series of functional groups that may react with any type of molecule containing the appropriate chemical functionality making possible the creation of one or more covalent links between particle and said molecule.

In this invention the term *fluid* is used without distinction to name gases or liquids. In the case of liquids it includes simple liquids, mixtures, solutions, suspensions, emulsions, liquidized solids, etc.

With the term *microjet* we refer to the capillary filament obtained from the flow focussing of the internal fluid inside the pressurized chamber that exits to the outside through a hole performed in the chamber. This term includes jets with nano and micrometric diameter and of different composition.

### Description of the invention

The aim of this invention is a procedure for the production of particles with labile molecules, peptides and proteins, cells, microorganisms, etc. inside them. This procedure is based on a capillary focussing system, which combines the application of hydrodynamic forces and a specific geometry of the system, to produce the generation of a capillary microjet inside a laminar flow, which due to capillary instabilities, breaks into drops with a controlled size and a very narrow and reproducible distribution. After solidification of the droplets we obtain particles of nano and micrometric size, with spherical shape keeping the distribution of the initially produced drops.

The basic technology of the invention lies on a system for introduction of a first fluid inside a pressurized chamber by means of a second fluid, in a way that produces a stable microjet of the first fluid, which is the one that breaks into homogeneous drops. The first fluid can be a liquid or gas and the second fluid can be a gas or a liquid. When both fluids are liquids, they must be different enough one to another to allow the generation of a stable microjet of the first fluid moving from the feeding end to the exit point of the pressurized chamber to the environment.

It is the aim of this invention a Flow Focussing device where the fluid injected through a capillary needle is a liquid and the fluid pressurizing the chamber is a liquid or a gas. Besides it is included in this invention a Flow Focussing device for the production of multiple capillary microjets, wherein the fluid injected through the multiple feeding tubes is a liquid and the fluid pressurizing the chamber is a liquid or a gas.

It is another aim of this invention the use of a device whose design includes a plurality of feeding needles placed concentrically to each other, so that among the fluids injected through those feeding points at least one is a liquid, and the fluid pressurizing the chamber is a liquid or a gas, producing one single capillary microjet that will produce the particles. It is included in this invention the use of a device for the generation of multiple capillary microjets by means of the use of multiple fluid feeding tubes, each of them constituted by two or more concentric capillaries, so that among the fluids injected through them at least one from each feeding tube is a liquid, and the fluid pressurizing the chamber is a liquid or a gas, producing one single capillary microjet that will produce the particles.

It is included in this invention the application of any of the habitual systems that allow solidification of the produced drops without losing their initial characteristics, and with no coalescence processes, clogging, etc. which could alter the morphology and size distribution of the particles. Among these systems for solidification of the drops are included solvent elimination, ionic gelification, thermal gelification, etc.

It is also included in this invention the application of a procedure for obtaining particles where the microjet is generated inside a fluid in motion in a way that at the moment when the microjet breaks into similar drops of the predicted size an emulsion is produced. After extraction/evaporation of the solvent by means of any of the already known methods we obtain particles of nano- and micrometric size with spherical shape and a very narrow and reproducible size distribution, as predicted by Flow Focussing theory. According to this invention, the extraction/evaporation of the solvent takes place producing only a reduction in the volume of the particle due to the solvent elimination. This elimination happens rapidly with no coalescence phenomenon of the drops, clogging or similar taking place, thus keeping the relative size distribution of the drops and final particles. In some cases, sizes slightly bigger to those predicted are obtained, due to a slowing down and widening of the focussed flow microjet that takes place before it breaks up due to the deceleration undergone by the external jet of the focussing fluid.

Preferably final particles obtained should have diameters between 0,01 and 1000 µm, more preferably between 0,01-200 µm and most preferably between 0,01-80 µm. Final particles obtained should be equal in size with a relative standard deviation of 10 to 30 %, more preferably 3 to 10 % and most preferably 3 % or less.

As an option, it is included in this invention the possibility to apply periodical and controlled external perturbations (e.g. mechanical, acoustic, etc.) to one or more fluids in order to enhance even more the production of particles with a homogeneous size distribution. These perturbations must be uniform and controlled and their frequency will be determined by the characteristics of the generated microjet and the required final particle size.

Nature and composition of the fluids referred to in this invention will depend on the composition and structure of the particles and on the final application these particles are produced for. In this invention the term *fluid* is used without distinction to name gases or liquids. In the case of liquids it includes simple liquids, mixtures, solutions, suspensions, emulsions, liquidized solids, etc.

Taking into account the final composition and structure of the particle, we must use a combination of fluids that: - has the required properties for the generation of a stable capillary microjet by means of any of the capillary flow focussing devices included in this patent, and - generates a capillary microjet that breaks up producing a stable system which allows, after solidification of the drop, obtaining particles of the predicted size and with a homogeneous size distribution.

It is aim of this invention that, when the production of an emulsion takes place, the device for the generation of drops is immerse in a fluid that will constitute the external phase of the emulsion so the drops don't undergo any deformation process during the generation of the emulsion. This fluid is a liquid that can have aqueous or organic nature, and whose properties are different enough from the fluid or fluids constituting the drops in order to produce an emulsion with droplets size equally in size to those produced by the dissociation of the capillary jet. Also, the fluid wherein the device is immersed can have substances in solution which enhance the generation and maintenance of uniformity and homogeneity of the emulsion during the drop solidification process for obtaining the particle (surfactant, emulsifying, tensioactive, etc.). Additionally, the fluid wherein the emulsion generates is in motion.

It is included in this invention a procedure for the generation of emulsions with very diverse characteristics according to the nature of the fluids in use (e.g. w/o, o/w, o/o, w/o/w, w/o/o, etc.).

The particles can be produced in different materials, including but not limited to polymers, silica, metal, ceramics, etc. This invention includes preferably polymeric materials. Polymers can be synthetic or natural, soluble in water or in organic solvents. An aim of this invention are fluids containing polymeric materials among which we can include, without limiting this invention: polyalcohols, polyacetals, polyethers, polyesters (such as polylactic acid, polyglycolic acid, poly(caprolactone) and similar ones and their copolymers), polyorthoesters, polyanhydrides (such as polysebacic acid, polyfumaric acid, poly(carboxyphenoxy propane), poly(carboxyphenoxy hexane) and similar ones and their copolymers), polyaldehydes, polyketones, polycarbonates, poly(iminocarbonates), polyamides, polyimide, polyacrylates and their derivates and copolymers, poly(cyancrilates), polyurethanes, polystyrenes, polychlorides, polyfluorides, polyvinyl derivates, polyolefins, polyphosphates, poly(organic phosphacens), poly(anhydrides-co-imides), polysaccharides, and carbohydrates derivates, poly(aminoacid), polymers derived from macromolecules, and all derivates of the ones mentioned above and their copolymers.

It is an aim of the invention that polymeric materials used in the production of particles have functional reactive groups that may react with any type of molecule containing the appropriate chemical functionality making possible the creation of one or more covalent bonds between particle and molecule. Preferably it is aim of this invention that those reactive groups are located in the surface of the particle directed to the outer surface. It is included in this invention that among the molecules linked to the surface are included, but not limited to, molecules of biological interest, preferably peptides, oligonucleotides, nucleic acids, PNAs, LNAs, proteins, glycoproteins, lipids, phospholipids, carbohydrates, oligosaccharides and mixtures of those.

It is also included in this invention a method for producing particles wherein the fluid constituting the matrix of the particle may have covalently linked molecules of biological interest exposed to the external environment of the particle.

Besides the main components that will constitute the particle matrix or matrixes (in the case of multilayer capsules), fluids can be composed of other compounds and labile substances that need to be protected during all or some of the following stages: formulation, distribution, storage and/or release of said substances. Among these substances are included, but not limited to: drugs and compounds with therapeutic and/or prophylactic activity, unstable molecules and compounds, peptides and proteins, microorganisms, cells, biomolecules, etc. individually or as a mixture of several of those.

In addition it is an aim of this invention that the particles containing labile material inside them have in their surface functional reactive groups able to create covalent bonds between the particle and other molecules. Additionally, this invention has as preferred procedure the one by means of which the generation of the particle with a reactive surface and the encapsulation of the fragile material takes place in one single step, using one of the devices described and included in this invention.

### METHOD FOR ACHIEVEMENT OF THE INVENTION

### EXAMPLE 1

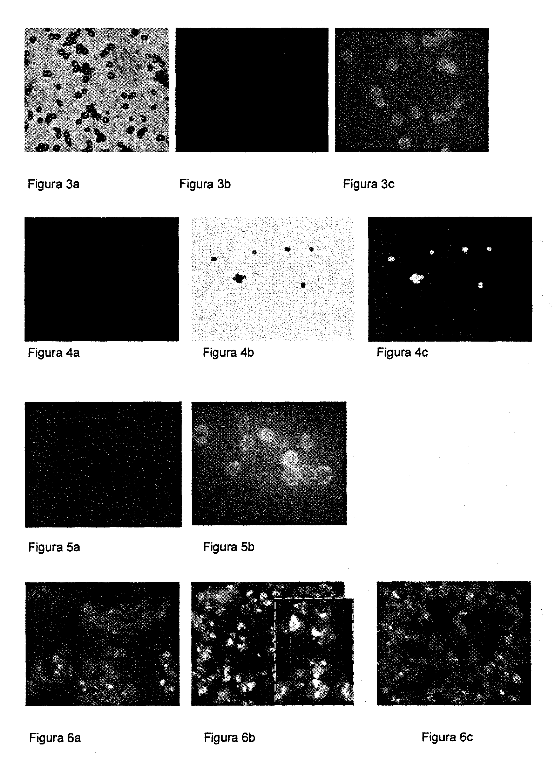

Encapsulation of BSA marked with fluorescein in alginate microparticles (Fig. 3)

The following example shows the procedure for encapsulation of BSA marked with fluorescein (BSA-Flu) in 11 micron alginate microparticles using the Flow Focussing technology in its liquid-gas configuration. The generation of the particles takes place using a capillary flow focussing device (D = 350 µm) with one single feeding tube (Do = 200; µm H = 125 µm). The device for capillary flow focussing is integrated in a spray-dryer LabPlant SD-Basic. Through the feeding tube we inject a BSA-Flu aqueous solution (0.17% w/v) in 1,7% w/v sodium alginate (Sigma) with 10 mL/h flow rate. The chamber is pressurized at 300 mbar by means of the introduction of a continuous gas flow. The drops are dried in the spray dryer at a temperature of 87°C and collected at the end of the process as dry particles.

The analysis of the microparticles was made using an optical microscope (Leica DM LS) and an image editor program (d average 10,75 µm, DS 0,71) and a fluorescency microscope (Leica DMR) (Fig.3a, 3c).

### EXAMPLE 2

Encapsulation of GFP in alginate microparticles (Fig. 4)

The following example shows the procedure for encapsulation of green fluorescent protein (GFP) in 11 micron alginate microparticles using the Flow Focussing technology in its liquid-gas configuration. It uses the same device for capillary flow focussing as in example 1. We prepare a GFP aqueous solution (0,04% w/v) in 1,7% w/v sodium alginate and the same procedure for production of microparticles as described in example 1 is followed.

The analysis of the microparticles was made using an optical microscope and an image editor program (d average 11,16 µm, DS 1,36) and a fluorescency microscope (Fig.4b, 4c).

### EXAMPLE 3

Encapsulation of BSA marked with fluorescein in polystyrene microparticles

The following example shows the procedure for encapsulation of BSA marked with fluorescein (BSA-Flu) in 13 micron polystyrene microparticles using the Flow Focussing technology in its liquid-liquid configuration. The generation of the emulsion takes place using a capillary flow focussing device (D = 100 µm) with one single feeding tube (Do = H = 150 µm). The device for capillary flow focussing is immersed in a 1 % w/v PVA aqueous solution under stirring. On a 2 mL 4 % w/v solution of polystyrene (Aldrich, Mw =4.000-200.000) in dichloromethane (Aldrich), we add drop by drop, and under continuous stirring, 0,140 mL of a 0,5% w/v solution of BSA marked with fluorescein in EtOH (Panreac Chemistry). This solution is injected through the feeding tube with 1 mL/h flow rate. The chamber is pressurized by means of the introduction of water with a 3 mL/min continuous flow rate. The generated o/w emulsion is kept under stirring during 16 h at room temperature so the extraction/evaporation of the solvent takes place. Solid particles are centrifuged (Orto Alresa mod. Digicen 20, 4.000 rpm, 10 min), washed three times in water, lyophilize and stored at 4 °C.

The analysis of the microparticles was made using an optical microscope (Leica DM LS) and an image editor program (d average 13,27 µm, DS 4,57) and a scanning electron microscope (Philips XL30) (Fig.5b).

### EXAMPLE 4

### Encapsulation of bacteria E. Coli in alginate microparticles

The following example shows the procedure for encapsulation of a modified GFP producer E. Coli strain in 11 micron alginate microparticles using the Flow Focussing technology in its liquid-gas configuration. It uses the same device for capillary flow focussing as in example 1. We add different amounts of an E.Coli suspension (4E8 c.f.u/mL) in NaCl (Sigma, 1% w/v) to 15 mL of a 2% w/v sodium alginate aqueous solution in order to generate suspensions with different bacteria concentrations (Table 1). The suspension is homogenized for 5 minute and the same procedure for microparticle production as described in example 1 is followed, this time the drop drying temperature is 99°C.

The analysis of the microparticles was made using an optical microscope (Leica DM LS) and an image editor program (Table 1) and a scanning electron microscope (Philips XL30) (Fig.6a-c).

**Table 1. Size distribution of microparticles with E. Coli based on the initial bacteria concentration.**

| **E. Coli concentration** | **d _{average}** (µm) | **DS** (µm) |
|---|---|---|
| 2·10⁸ cfu/gr | 10,3 | 1,16 |
| 8·10⁸ cfu/gr | 11,15 | 1,48 |
| 1,3·10⁹ cfu/gr | 11,51 | 1,60 |

## Claims

1. Procedure for the production of particles in the micro and nanometric range with encapsulated labile products **characterized by** that it is carried out with a Flow Focusing device, following by a solidification step to turn the drops into particles.

2. Procedure for the production of particles in the micro and nanometric range with encapsulated labile products, according to claim 1, **characterized by** that at least one focusing fluid must be a liquid.

3. Procedure for obtaining particles in the micro- and nanometric range with encapsulated labile products according to claims 1-2 **characterized by** that the focussed liquid fluids can be simple liquids, mixtures, solutions, suspensions, emulsions, liquidized solids, chosen in order to generate a stable microjet of the fluid or fluids focussed by the focussing fluid.

4. Procedure for obtaining particles in the micro- and nanometric range with encapsulated labile products according to claims 1 - 3 **characterized by** that at least one of the focussed liquid fluids used must contain the labile compounds, which will be encapsulated within the particles containing them.

5. Procedure for obtaining particles in the micro- and nanometric range with encapsulated labile products according to claims 1 - 4 **characterized by** that at least one of the focussed liquid fluids used includes polymeric materials, silica, metals or ceramics, which constitute the matrix of the particles.

6. Procedure for obtaining particles in the micro- and nanometric range with encapsulated labile products according to claim 5 **characterized by** that at least one of the focussed liquid fluids is preferably a solution, mixture, suspension and/or an homogeneous emulsion of a polymeric material.

7. Procedure for obtaining particles in the micro- and nanometric range with encapsulated labile products according to claim 6 **characterized by** that the injected polymeric material can be synthetic or natural, soluble in water or in organic solvents.

8. Procedure for obtaining particles in the micro- and nanometric range with encapsulated labile products according to claim 7 **characterized by** that the polymeric material is selected preferably among the following: polyalcohols, polyacetals, polyethers, polyesters (such as polylactic acid, polyglycolic acid, poly(caprolactone) and similar ones and their copolymers), polyorthoesters, polyanhydrides (such as polysebacic acid, polyfumaric acid, poly(carboxyphenoxy propane), poly(carboxyphenoxy hexane) and similar ones and their copolymers), polyaldehydes, polyketones, polycarbonates, poly(iminocarbonates), polyamides, polyimide, polyacrylates, poly(cyancrilates), polyurethanes, polystyrenes, polychlorides, polyfluorides, polyvinyl derivates, polyolefins, polyphosphates, poly(organic phosphacens), poly(anhydrides-co-imides), polysaccharides, and carbohydrates derivates, poly(aminoacid) and polymers derived from macromolecules.

9. Procedure for obtaining particles in the micro- and nanometric range with encapsulated labile products according to claim 8 **characterized by** that the used polymeric materials have functional reactive groups that may react with any type of molecule containing the appropriate chemical functionality making possible the creation of one or more covalent bonds between particle and said molecule.

10. Particles in the micro- and nanometric range with encapsulated labile products generated by means of a procedure according to claims 1 - 9 **characterized by** that the labile products included in the invention present thermal instability, photosensitivity, enzymatic, microbiology and chemical sensitivity.

11. Particles in the micro- and nanometric range with encapsulated labile products according to claim 10 **characterized by** that the labile products are organic molecules, drugs and compounds with therapeutic and/or prophylactic activity, peptides, proteins and protean complexes, oligonucleotides, nucleic acids, PNAs, LNAs, DNAs, RNAs, viruses and related , organelles, cells, microorganisms and mixtures of those.

12. Particles in the micro- and nanometric range with encapsulated labile products according to claims 10 - 11 **characterized by** that the matrix of said particles includes polymeric materials, silica, metals or ceramics.

13. Particles in the micro- and nanometric range with encapsulated labile products according to claim 12 **characterized by** that the matrix is preferably a solution, blending, suspension and/or an homogeneous emulsion of a polymeric material.

14. Particles in the micro- and nanometric range with encapsulated labile products according to claim 13 **characterized by** that the polymeric material can be synthetic or natural, soluble in water or in organic solvents.

15. Particles in the micro- and nanometric range with encapsulated labile products according to claim 14 **characterized by** that the polymeric material is selected preferably among the following: polyalcohols, polyacetals, polyethers, polyesters (such as polylactic acid, polyglycolic acid, poly(caprolactone) and similar ones and their copolymers), polyorthoesters, polyanhydrides (such as polysebacic acid, polyfumaric acid, poly(carboxyphenoxy propane), poly(carboxyphenoxy hexane) and similar ones and their copolymers), polyaldehydes, polyketones, polycarbonates, poly(iminocarbonates), polyamides, polyimide, polyacrylates, poly(cyancrilates), polyurethanes, polystyrenes, polychlorides, polyfluorides, polyvinyl derivates, polyolefins, polyphosphates, poly(organic phosphacens), poly(anhydrides-co-imides), polysaccharides, and carbohydrates derivates, poly(aminoacid), polymers derived from macromolecules, and all derivates of the ones mentioned above and their copolymers.

16. Particles in the micro- and nanometric range with encapsulated labile products according to claims 13 - 15 **characterized by** that the applied polymeric materials have functional reactive groups that may react with any type of molecule containing the appropriate chemical functionality making possible the formation of one or more covalent bonds between particle and molecule.

17. Particles in the micro- and nanometric range with encapsulated labile products according to claim 16 **characterized by** that those reactive groups of the materials used are located in the surface of the drops exposed to the external environment.

18. Particles in the micro- and nanometric range with encapsulated labile products according to claims 12 - 15 **characterized by** that the material that will constitute the particle matrix can have covalently linked molecules of biological interest that will be exposed to the outside of the particles, preferably peptides, oligonucleotides, nucleic acids, PNAs, LNAs, proteins, glycoproteins, lipids, phospholipids, carbohydrates, oligosaccharides and mixtures of those.

19. Particles in the micro- and nanometric range with encapsulated labile products according to claims 10 18 **characterized by** that the particles have a diameter between 0,01 and 1000 micron.

20. Particles in the micro- and nanometric range with encapsulated labile products according to claims 10 - 19 **characterized by** that the particles have a size distribution with a relative standard deviation of 10 to 30 %, preferably 3 to 10 % and more preferably less than 3 %.

21. Particles in the micro- and nanometric range with encapsulated labile products according to claims 10 - 20 **characterized by** that the particles obtained can be solid, hollow or porous.

22. Particles in the micro- and nanometric range with encapsulated labile products according to claim10 - 21 **characterized by** that the particles have a homogeneous matrix.

23. Particles in the micro- and nanometric range with encapsulated labile products according to claims 10 - 21 **characterized by** that the particles have a non homogeneous matrix constituted by deferent concentric layers.

24. Particles in the micro- and nanometric range with encapsulated labile products according to claims 10 - 23, in which the particles have in their surface functional reactive groups that can create covalent bonds with molecules of biological interest, preferably peptides, oligonucleotides, nucleic acids, PNAs, LNAs, proteins, glycoproteins, lipids, phospholipids, carbohydrates, oligosaccharides and mixtures of those.
